# EUROPEAN PATENT SPECIFICATION

(11) **EP 2 662 671 B1**
(45) Date of publication and mention of the grant of the patent: **28.03.2018**
(21) Application number: 12167108.5
(22) Date of filing: 08.05.2012
(51) Int. Cl.: G01F 11/02, A61M 5/168, B01L 3/02, G01N 35/10, A61M 5/142, G01N 35/00, B01L 3/00

(54) **Cartridge for dispensing a fluid**
Kartusche zur Ausgabe einer Flüssigkeit
Cartouche pour distribuer un fluide

(43) Date of publication of application: 13.11.2013
(73) Proprietor: Roche Diagnostics GmbH, 68305 Mannheim (DE); F. Hoffmann-La Roche AG, 4070 Basel (CH)
(72) Inventor: Böhm, Christoph, 68519 Viernheim (DE); Kupser, Peter, 81379 München (DE); Oranth, Norbert, 79279 Vörstetten (DE); Spinke, Jürgen, Dr., 64653 Lorsch (DE)
(74) Representative: Richardt Patentanwälte PartG mbB

(56) References cited:
- EP-A1- 1 987 890
- WO-A1-2010/044788
- US-A- 5 441 173
- US-A1- 2009 176 314

## Description

### Field of the invention

The invention relates to cartridges for dispensing a fluid. The invention further relates to an automatic analyzer for dispensing the fluid using the cartridge.

### Background and related art

In medical laboratories, in vitro diagnostics are commonly performed on biological samples such as blood, urine, blood plasma and saliva. Such tests may be performed manually using pipettes or maybe performed using an automatic analyzer.

Automatic analyzers may automatically add reagents to the biological sample and may measure one or more parameters of the biological sample during analysis. Automatic analyzers are known in the prior art. For example, European patent EP 1 959 257 A2 discloses an automatic analyzer including a reagent cassette holding mechanism for holding a plurality of reagent cassettes.

United States patent US 7,955,302 B2 discloses a dosing device for an infusion system comprising a dosing unit having a variable volume and at least one opening in fluid connection with the variable volume.

International patent application WO 2010/044788 A1 discloses a fluid ejection cartridge for a fluid ejection device. The cartridge includes a print head, having a plurality of fluid ejection nozzles, a fluid reservoir, configured to hold a fluid to be ejected from the print head, and a selectively breachable isolator mechanism, separating the fluid reservoir and the print head.

European patent application EP 1 987 890 A1 discloses a liquid material discharge device which can remove bubbles in a liquid material supplied to the device, can ensure a stable discharge amount of the liquid material, and can reduce the weight of a plunger section. The liquid material discharge device comprises a liquid material supply section for supplying the liquid material to be discharged, a measuring section having a measuring hole and a plunger sliding on an inner wall surface of the measuring hole to suck the liquid material into the measuring hole and to discharge the liquid material, a discharge section having a discharge port for discharging the liquid material, a valve section for changing over communication between the liquid material supply section and the measuring section and communication between the measuring section and the discharge section, and a debubbling mechanism provided in a flow path running from the liquid material supply section to the measuring section. The debubbling mechanism includes a first flow path communicating with the liquid material supply section side, a second flow path communicating with the measuring section side, and a body for making the first flow path and the second flow path communicate with each other, the body having a greater width than the first flow path. A discharge port of the first flow path is located above a suction port of the second flow path.

### Summary

The invention provides for a cartridge for dispensing fluid and an automatic analyzer in the independent claims. Embodiments are described in the dependent claims.

The present invention provides for a cartridge for dispensing a fluid. The cartridge comprises a rotary valve which may be moved in a circular fashion to position a pumping chamber conduit coming from a pumping chamber. Rotation of the rotary valve enables the pumping chamber conduit to be connected to one of a variety of other conduits. The pumping chamber is formed by a cavity within the rotary valve and by a plunger which is operable for changing the volume of the pumping chamber.

The cartridge comprises a reservoir for storing the fluid and an outlet conduit for dispensing a fluid. A reservoir conduit connects the reservoir with the rotary valve. In some embodiments, the outlet conduct conduit connects an outlet nozzle to the rotary valve. As the rotary valve is moved in different positions the pumping chamber conduit can be positioned at either the reservoir conduit or the outlet conduit. The rotary valve and the plunger are able to be operated or actuated independently of each other. Embodiments of this cartridge may have the advantage that they can be operated such that the cartridge does not lose any fluid or that the fluid loss due to priming can be reduced.

A controller as used herein encompasses a device, machine, or apparatus for controlling the operation and/or function of one or more other devices. Examples of a controller may include, but are not limited to: a computer, a processor, an imbedded system or controller, a programmable logic controller, and a microcontroller. A 'computing device' or 'computer' as used herein encompasses to any device comprising a processor. A 'processor' as used herein encompasses an electronic component which is able to execute a program or machine executable instruction.

A 'computer-readable storage medium' as used herein encompasses any tangible storage medium which may store instructions which are executable by a processor of a computing device. The computer-readable storage medium may be referred to as a computer-readable non-transitory storage medium.

'Computer memory' or 'memory' is an example of a computer-readable storage medium. Computer memory is any memory which is directly accessible to a processor or other controller. 'Computer storage' or 'storage' is an example of a computer-readable storage medium. Computer storage is any non-volatile computer-readable storage medium.

A 'user interface' as used herein is an interface which allows a user or operator to interact with a computer or computer system.

A 'hardware interface' as used herein encompasses a interface which enables a processor or other controller to interact with and/or control an external computing device and/or apparatus. A hardware interface may allow a processor to send control signals or instructions to an external computing device and/or apparatus. The hardware interface may enable the processor or other controller to receive sensor data and control the dispensing of the fluid. The hardware interface may be used to form a closed control loop in some embodiments.

In one aspect the invention provides for a cartridge for dispensing fluid. The cartridge comprises a cartridge body, a reservoir for storing a fluid, and a reservoir conduit for connecting the reservoir with the rotary valve, an outlet conduit for dispensing the fluid. The cartridge body comprises a cylindrical space and rotary valve. The rotary valve comprises a cylindrical portion adapted to fit into the cylindrical space. The rotary valve comprises further a pumping chamber for pumping the fluid. The pumping chamber comprises a pumping chamber conduit which is formed in a wall of the cylindrical portion. The rotary valve is operable for rotating the pumping chamber conduit. The pumping chamber conduit is connected with the pumping chamber. In other words there is a rotary valve which has a pumping chamber conduit connected to a pumping chamber within it. By rotating the rotary valve the pumping chamber conduit can be rotated into different positions thereby allowing the pumping chamber to be connected to reservoir conduit or the outlet conduit.

The cartridge body further comprises a plunger operable for changing the volume of the pumping chamber. The cylindrical potion comprises a cavity. The pumping chamber is formed by the cavity and the plunger. The rotary valve and the plunger are operable for being actuated independently. In other words the plunger and the rotary valve are able to be operated such that the plunger can be used to change the volume of the pumping chamber without affecting the position of the rotary valve and vice versa. This may enable a larger set of pumping actions by the pumping chamber. The cartridge further comprises a reservoir for storing the fluid. The cartridge further comprises a reservoir conduit for connecting the reservoir with the rotary valve.

This embodiment may have the advantage that a large variety of pumping actions can be performed with the pumping chamber by controlling the rotational position of the rotary valve and properly operating the plunger. For instance the rotary valve may be positioned such that the pumping chamber conduit is connected to the reservoir conduit. In this case the plunger may be used to either withdraw fluid from the reservoir into the pumping chamber or may be used to pump the fluid from the pumping chamber back into the reservoir.

The present embodiment may enable other types of actions using the pumping chamber. For instance when the pumping chamber conduit is aligned or connected with the reservoir conduit the plunger may be repeatedly used to increase and decrease the volume of the pumping chamber. This may enable the fluid within the reservoir to be mixed. Also the ability to put the fluid back into the reservoir may reduce the amount of fluid that is wasted.

This embodiment may also enable a so called reduced waste priming or non-waste priming function of the pumping chamber whereby none or possibly only a very small amount of the fluid is wasted or discarded when fluid is pumped out through the outlet conduit. For instance when the pumping chamber conduit is connected with the outlet conduit the plunger may be used to decrease the volume of the pumping chamber and thereby force or dispense fluid out through the outlet conduit. During the process of doing this there may be fluid within the outlet conduit which does not exit the outlet conduit. After the correct amount of fluid has been dispensed the plunger may then be used to increase the volume of the pumping chamber thereby withdrawing fluid that may remain within the outlet conduit back into the pumping chamber. Fluid could then be held within the pumping chamber or if the rotary valve is rotated into alignment with the reservoir conduit the fluid which was previously within the outlet conduit may be pumped back into the reservoir.

The rotary valve may also provide a means of preventing fluid from accidentally leaking from the reservoir. For instance the rotary valve may be able to be rotated in some embodiments to a position where it is neither aligned with the outlet conduit nor with the reservoir conduit. This may prevent fluid and/or gas from exiting from the outlet conduit and/or from fluid and/or gas in a reservoir leaking or draining into the pumping chamber.

In another embodiment the cartridge further comprises an outlet nozzle connected to the outlet conduit. An outlet nozzle as used herein encompasses a nozzle design to minimize the waste of fluid and may enables drops to cleanly drip during the dosing process. For instance in a simple tube a drop of the fluid may hang outside the nozzle after the plunger is used to decrease the volume of the pumping chamber. The shape or function of the outlet nozzle can be designed to reduce the chances of a drop of the fluid hanging on it. For instance the outlet nozzle may have a so called duckbill shape and be a duckbill nozzle.

In other embodiments the cartridge may have additional reservoirs and additional reservoir conduits which enable the pumping chamber to be connected to these additional reservoirs. Typically a cartridge may contain only a single fluid or reagent. In some embodiments this may be a diluent that is used or required for various tests.

There may also be multiple reservoirs which may be each connected to a conduit accessible to the pumping chamber conduit at a particular rotational position of the rotary valve.

For example for many clinical tests there may be two reservoirs and for immunoassays there may be two or three different fluids within different reservoirs. In some variations of this embodiments the cartridge may have multiple pumping units, with each of the pumping units being connected to one or more reservoirs via its rotary valve. In this way the immunoassays are dispensed using separate pumping units and they are not mixed by the pumping process.

In another embodiment the cartridge further contains a return conduit connected to the reservoir. The pumping chamber conduit is operable for receiving fluid from a first portion of the reservoir. The return conduit is operable for returning fluid to a second portion of the reservoir. The rotary valve is further operable for rotating the pumping chamber conduit to connect to the return conduit. This embodiment may be beneficial because it may for instance reduce the effect of potentially occurring gas bubbles when fluid is returned to the reservoir.

For instance fluid could be drawn from the reservoir when the rotary valve is rotated such that the pumping chamber conduit is in alignment with the reservoir conduit. After a certain amount of the fluid has been dispensed through the outlet conduit the rotary valve might be rotated into such a position such that the pumping chamber conduit is in alignment with the return conduit. The reservoir conduit may draw fluid from one portion of the reservoir and the return conduit is used to return the fluid to a different portion of the reservoir. For instance the two locations of the reservoir conduit and the return conduit could be far enough away that it is unlikely that bubbles which enter the reservoir through the return conduit are drawn into the reservoir conduit when fluid is drawn from the reservoir into the pumping chamber.

In another embodiment the cartridge further comprises a secondary reservoir. The cartridge further comprises a secondary reservoir conduit. The rotary valve is further operable for rotating the pumping chamber conduit to connect to the secondary reservoir conduit. This embodiment may be beneficial because it may enable a second or distinct fluid to be stored and dispensed using the cartridge, it may also enable waste fluid to be disposed of in the secondary reservoir.

It should be noted that additional reservoirs may be added to the cartridge by adding a third reservoir and a third reservoir conduit, a fourth reservoir and a fourth reservoir conduit, and so on so that any number of reservoirs and reservoir conduits may be added to the cartridge.

In another embodiment the cartridge further comprises a connecting conduit. The connecting conduit is operable for transporting fluid between the secondary reservoir and the reservoir. This embodiment may be beneficial because the connecting conduit may enable the secondary reservoir to be used as a place to deposit fluid in order to return it to the reservoir.

In another embodiment the cartridge comprises a membrane blocking the connecting conduit. The membrane is permeable to the fluid. This embodiment may be beneficial because it may provide a means of filtering fluid or blocking of gas bubbles when fluid is returning from the secondary reservoir into the reservoir.

In another embodiment the secondary reservoir comprises a bubble guiding structure. A bubble guiding structure as used herein encompasses a structure which is used to guide a gas bubble to a predetermined location in a reservoir or towards a vent. In some implementations the bubble guiding structure may allow fluid to pass around the bubble as it is moving through the reservoir. For instance a bubble structure may be a set of ridges which are used to position and guide a bubble. The structures and ridges may be spaced close enough together such that the surface tension of the fluid prevents the bubble from going into regions which allow the fluid to go around the bubble. This may be beneficial because if the bubble is not properly confined the bubble may get stuck at a particular position in the secondary reservoir and not allowed to go to the top of the secondary reservoir or in the case where there is a connecting conduit to allow the fluid to return to the reservoir.

In another embodiment the secondary reservoir comprises a vent. A vent as used herein is a structure which enables air bubbles or other gas volumes to enter or leave the cartridge. Alternatively, the reservoir comprises such a vent or both the reservoir and the second reservoir comprise such vents

In another embodiment the vent is covered or sealed with a filter. The filter is operable for sealing the fluid in the cartridge. The filter may be hydrophobic in some embodiments. In some embodiments the gas filter may have micropores to only let gas through, but no liquids. In some embodiments the filter may be, but is not limited to: a porous form of polytetrafluoroethylene, carbon fibers, carbon fibers coated with PTFE, carbon nanotubes, polymer fibers, or fluropolymer fibers

In another embodiment the fluid comprises magnetic beads.

In another embodiment the fluid comprises latex beads.

In another embodiment the fluid comprises a blood grouping reagent.

In another embodiment the fluid comprises an immune reagent.

In another embodiment the fluid comprises an antibody.

In another embodiment the fluid comprises an enzyme.

In another embodiment the fluid comprises a recombinant protein.

In another embodiment the fluid comprises a virus isolate.

In another embodiment the fluid comprises a virus.

In another embodiment the fluid comprises a biological reagent.

In another embodiment the fluid comprises a solvent.

In another embodiment the fluid comprises a diluent.

In another embodiment the fluid comprises a dispersion.

In another embodiment the fluid comprises nanoparticles.

In another embodiment the fluid comprises a protein.

In another embodiment the fluid comprises a salt.

In another embodiment the fluid comprises a detergent.

In another embodiment the fluid comprises a nucleic acid.

In another embodiment the fluid comprises an acid.

In another embodiment the fluid comprises a base.

In another embodiment the cartridge further comprises a sensor operable for metering fluid dispensed by the outlet conduit. For instance this sensor may be a capacitive or optical sensor.

In another embodiment the cartridge further comprises a coupling assembly for attaching the rotary valve and the plunger to an actuator assembly. This embodiment may be beneficial because it may enable the rotary valve and the plunger to be conveniently connected to an actuator. The coupling assembly in some embodiments may enable the rotary valve and the plunger to be actuated independently by the actuator assembly.

In some embodiments it may be possible to have a cartridge with its own actuator. In this case the cartridge further comprises an actuator. In some cases the actuator may be connected to the coupling assembly or the actuator may be designed or operable for directly actuating the rotary valve and the plunger independently.

A pumping unit as used here encompasses the rotary valve and plunger for pumping the fluid. When installed into an automatic analyzer there may be one actuator per pumping unit or there may be one actuator which is moved and used to actuate all of the cartridges within the automatic analyzer. In this case there may be a mechanism for moving the relative positions between the cartridge and the single actuator. There may also be an actuator for a group of cartridges.

For example there may be different configurations for the cartridge. In some embodiments the cartridge may have a single pumping unit. This single pumping unit may have conduits connected to different reservoirs. This may enable the cartridge to pump different types of fluids from the same cartridge. In another example the cartridge may have multiple pumping units, with each of the pumping units being connected to one or more reservoirs via its rotary valve.

In another embodiment the rotary valve comprises a cylindrical portion. The pumping chamber is a cavity within the rotary valve. The pumping chamber is formed by the cavity and the plunger. The cartridge comprises a cartridge body with a cylindrical space for receiving the cylindrical portion. The rotary valve is operable for rotating within the cylindrical space.

In another embodiment the reservoir conduit and the outlet conduit are located on the cylindrical space. The pumping chamber conduit is located on the cylindrical portion.

In another embodiment the cartridge comprises multiple pumping units.

In another embodiment the cartridge comprises multiple reservoirs.

In another embodiment the multiple reservoirs are filled with different fluids.

In another aspect the invention provides for an automatic analyzer for holding a cartridge according to an embodiment of the invention. An automatic analyzer as used herein encompasses a system for automatically analyzing a biological sample. The automatic analyzer comprises an actuator assembly operable for linear actuation of the plunger and for rotational actuation of the rotary valve. The actuator assembly is further operable for actuating the plunger and the rotary valve independently. The automatic analyzer further comprises a controller for controlling the operation of the actuator assembly.

In some embodiments the automatic analyzer may be adapted for holding multiple cartridges according to an embodiment of the invention. In this case there may be a mechanism for providing relative movement between the cartridges and a reaction tube / cuvette. There may be one actuator per pumping unit or there may be one actuator used for multiple cartridges. In this case there may be a mechanism or a robotic system for providing relative movement between the cartridge and the actuator. There may also be embodiments where there are multiple actuators each used for a group of cartridges. The group of cartridges could be predetermined or the group of cartridges may be determined on the fly. Alternatively, multiple actuators may be used for a cartridge or a group of cartridges, e.g. for different purposes like pre-dispensing or post-dispensing actions.

In another embodiment the automatic analyzer comprises the cartridge.

In another embodiment the controller is operable for controlling the actuator assembly to rotate the pumping chamber conduit to connect with the reservoir conduit by rotating the rotary valve. The controller is further operable for controlling the actuator assembly to fill the pumping chamber by increasing the volume of the pumping chamber with the plunger. The controller is further operable for controlling the actuator assembly to rotate the pumping chamber conduit to connect with the outlet conduit by rotating the rotary valve. The controller is further operable for controlling the actuator assembly to pump the fluid through the outlet conduit by decreasing the volume of the pumping chamber with the plunger. This embodiment may be beneficial because it provides a method of pumping fluid through the outlet conduit.

In another embodiment the controller is operable for controlling the actuator assembly to receive the fluid from the outlet conduit by increasing the volume of the pumping chamber with the plunger.

In another embodiment the controller is operable for controlling the actuator assembly to rotate the pumping chamber conduit to connect with the reservoir conduit by rotating the rotary valve. The controller is further operable for controlling the actuator assembly to return the fluid to the reservoir by decreasing the volume of the pumping chamber with the plunger. This embodiment may be advantageous because it provides operation of the pump without priming. 100% or nearly 100% of the fluid may be used.

In another embodiment the controller is operable for controlling the actuator assembly to rotate the pumping chamber conduit to connect with the reservoir conduit by rotating the rotary valve. The controller is further operable for controlling the actuator assembly to mix the fluid in the reservoir by repeatedly increasing and decreasing the volume of the pumping chamber with the plunger. In the case where the fluid contains beads or particles such as magnetic or latex beads this embodiment may be used to mix the fluid and its compounds.

In another embodiment the the cartridge comprises an outlet nozzle. The automatic analyzer further comprises a meniscus detector for detecting a meniscus of the fluid. The controller is operable for controlling the actuator to force fluid through the outlet nozzle. The controller is further operable for detecting the meniscus using the meniscus detector. The controller is further operable for controlling the actuator to halt the forcing of fluid through the outlet when the meniscus is in a predetermined location. This embodiment may be beneficial because it may enable more accurate and more precise dispensing of the fluid. This embodiment may be beneficial because if the meniscus is in the same place when the fluid dispensing starts then the dispensing of the fluid may be more accurate, more precise and/or more reproducible. The meniscus may be inside or outside the outlet nozzle. For instance the outlet nozzle may be a long tube-like structure and the meniscus may have a particular position within the tube. In other embodiments the meniscus may be formed by a drop of the fluid hanging from the outlet nozzle. In many applications, the meniscus is preferably positioned right at the orifice of the outlet nozzle.

In another embodiment the controller is further operable for controlling the actuator to force a predetermined volume of fluid through the outlet. In some embodiments the actuator may be controlled to force the predetermined volume of fluid through the outlet nozzle after the meniscus is in the predetermined location.

In another embodiment the meniscus detector is any one of the following: a capacitive sensor, an optical sensor and a camera. When the meniscus is inside of the nozzle a capacitive sensor may be used to detect the location of the meniscus. In case the nozzle is optically transparent an optical sensor may also be used to determine the location of the meniscus within the nozzle. If the meniscus extends beyond the nozzle then a capacitive sensor, an optical sensor or a camera may each be used to determine the location of the meniscus.

In another embodiment the automatic analyzer is operable to hold multiple cartridges. Each of the multiple cartridges is according to an embodiment of the invention.

In another embodiment the automatic analyzer further comprises the multiple cartridges.

The embodiment with the multiple cartridges may be implemented in a variety of ways. For example each pumping unit may have its own actuator assembly. This may be a parallel operation. In another example cartridges may be moved and put onto the same actuator assembly or an actuator assembly may be moved between different cartridges or even between different pumping units that are part of the same cartridge. In yet other embodiments there may be multiple actuators and cartridges are moved via a mechanical robotic system between these multiple actuators.

In another embodiment the automatic analyzer comprises a sensor or metering system operable for measuring or metering the dispensing of the fluid. The controller is operable for controlling the dispensing of the fluid in accordance with measurements or data received from the sensor or metering system. In other words the controller is operable for forming a closed loop control system with the sensor or metering system for controlling the dispensing of the fluid.

In another aspect the invention provides for a method of operating a cartridge according to an embodiment of the invention, wherein the cylindrical portion is configured to be rotated within the cylindrical space such that: the pumping chamber conduit can be connected with the reservoir conduit thereby closing the outlet conduit by the cylindrical portion and can be connected with the outlet conduit thereby closing the reservoir conduit by the cylindrical portion; and the plunger being configured to be actuated in a linear direction such that the volume of the pumping chamber can be increased to fill the pumping chamber and decreased to pump the fluid through the outlet conduit. The method comprises the steps of:
rotating the rotary valve to rotate the pumping chamber conduit to connect with the reservoir conduit; increasing the volume of the pumping chamber with the plunger to fill the pumping chamber; rotating the rotary valve to rotate the pumping chamber conduit to connect with the outlet conduit; decreasing the volume of the pumping chamber with the plunger to pump the fluid through the outlet conduit.

In another embodiment the method further comprises the step of increasing the volume of the pumping chamber with the plunger to retrieve the fluid from the outlet conduit.

In another embodiment the method further comprises the steps of: rotating the rotary valve to rotate the pumping chamber conduit to connect with the reservoir conduit; and decreasing the volume of the pumping chamber with the plunger to return the fluid to the reservoir.

In another embodiment the method further comprises the steps of: rotating the rotary valve to rotate the pumping chamber conduit to connect with the reservoir conduit; and repeatedly increasing and decreasing the volume of the pumping chamber with the plunger to mix the fluid in the reservoir.

### Brief description of the drawings

In the following embodiments of the invention are explained in greater detail, by way of example only, making reference to the drawings in which:
Fig.1 illustrates a cartridge and an actuator assembly according to an embodiment of the invention;
Fig. 2 illustrates how the cartridge may be used to pump fluid through the outlet conduit;
Fig. 3 illustrates a pumping method similar to that shown in Fig. 2 except additional steps are performed to remove fluid from the outlet conduit;
Fig. 4A and 4B illustrates how fluid can be pumped through the outlet conduit where the fluid is taken from the reservoir and then excess fluid from the outlet nozzle and outlet conduit is pumped into the secondary reservoir;
Fig. 5 illustrates an automatic analyzer according to an embodiment of the invention;
Fig. 6 illustrates a bubble guide according to an embodiment of the invention;
Fig. 7 illustrates an automatic analyzer according to a further embodiment of the invention; and
Fig. 8A, 8B, 8C, and 8D illustrate the operation of a cartridge using a meniscus detector.

### Detailed description

Like numbered elements in these figures are either equivalent elements or perform the same function. Elements which have been discussed previously will not necessarily be discussed in later figures if the function is equivalent.

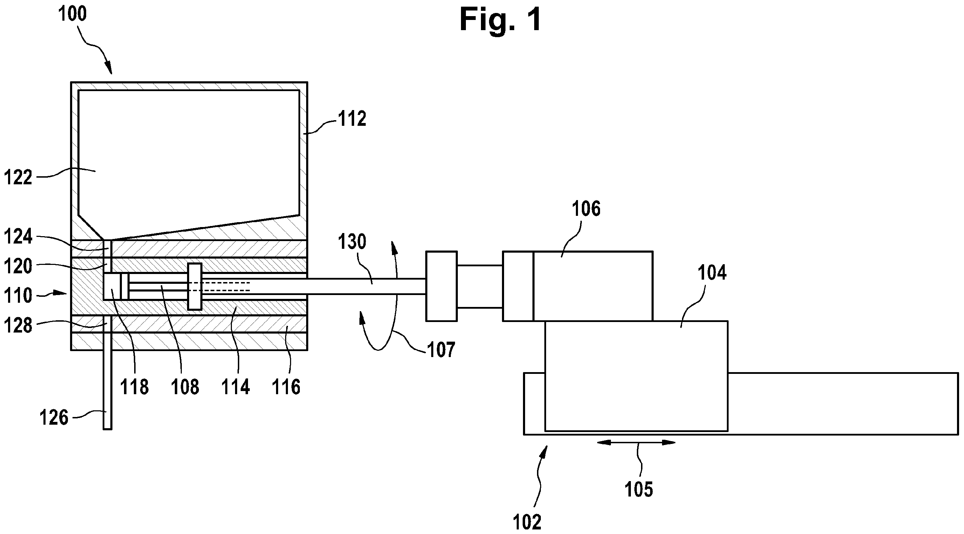

Fig. 1 illustrates a cartridge 100 and an actuator assembly 102 according to an embodiment of the invention. The actuator assembly 102 comprises a linear actuator 104 which is able to actuate in direction 105. The actuator assembly 102 further comprises a rotational actuator 106 able to actuate in the direction 107.

The cartridge 100 comprises a plunger 108 and a rotary valve 110. The cartridge 100 comprises a cartridge body 112 which has a cylindrical space 116. In this case the cylindrical space 116 is formed by a bearing material. The rotary valve 110 has at least a cylindrical portion 114 adapted to fit into the cylindrical space 116 of the cartridge body 112. The rotary valve 110 has a hollow space which forms a pumping chamber 118 which is formed by the hollow space and the plunger 108. The pumping chamber 118 has a pumping chamber conduit 120 which is formed in a wall of the rotary valve 110. The rotary valve 110 is operable for rotating within the cylindrical space 116 to position the pumping chamber conduit 120 at different angular positions.

The cartridge 100 further comprises a reservoir 122 for being filled with a liquid. It is not shown in Fig. 1 but the cartridge 100 may also comprise a vent for allowing gas to be vented into the reservoir 122. The cartridge 100 further comprises a reservoir conduit 124. The reservoir conduit 124 provides the reservoir 122 access to the pumping chamber conduit 120 when the pumping chamber conduit is in the correct rotational position. The cartridge 100 also comprises an optional outlet nozzle 126 for dispensing the fluid. The outlet nozzle 126 is connected to an outlet conduit 128. The outlet conduit 128 allows the pumping chamber 118 to dispense the fluid. The outlet conduit 128 in this embodiment is connected to the outlet nozzle 126 when the pumping chamber conduit 120 is in the correct rotational position. There is further shown a coupling assembly 130 which couples the actuator assembly 102 to the cartridge 100. The coupling assembly 130 is designed for being and actuating the piston 108 in the linear direction 105. The coupling assembly 130 is also adapted for being able to independently rotate the rotary valve 110. For instance there may be grooves cut into the rotary valve 110 and there may be a shape on the coupling assembly 130 which mates into the groove of the rotary valve 110. The example shown in Fig. 1 is only one way in which the rotary valve 110 and the piston 108 may be actuated. Other equivalent mechanisms may also be used to actuate and attach to the rotary valve 110 and the piston 108.

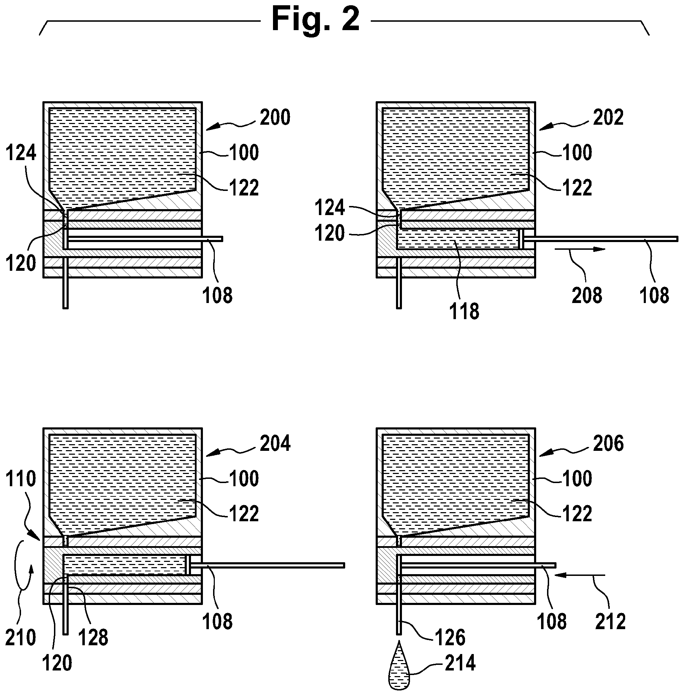

Fig. 2 illustrates four views 200, 202, 204, 206 of the cartridge 100. Fig. 2 illustrates how the cartridge 100 may be used to pump fluid through the outlet conduit128. In view 200 the pumping chamber conduit 120 is aligned with the reservoir conduit 124. The plunger 108 is fully depressed and the pumping chamber 118 has no volume or is extremely small. In this example the plunger 108 is fully depressed. However, fully depressing the plunger 108 is not a requirement for the operation. In the examples described herein the relative motion of the plunger is what is relevant. For example, depressing the plunger 108 causes the volume of the pumping chamber to decrease and this forces the fluid through the outlet conduit.

Next in view 202 the plunger is withdrawn in direction 208. This causes fluid from the reservoir 122 to enter the pumping chamber 118. Next in view 204 the rotary valve 110 is rotated 210 such that the pumping chamber conduit 120 is aligned with the outlet conduit 128. The pumping chamber 118 is now isolated from the reservoir 122. Next in view 206 the plunger 108 is depressed in direction 212 and fluid 214 exits via the outlet conduit 128.

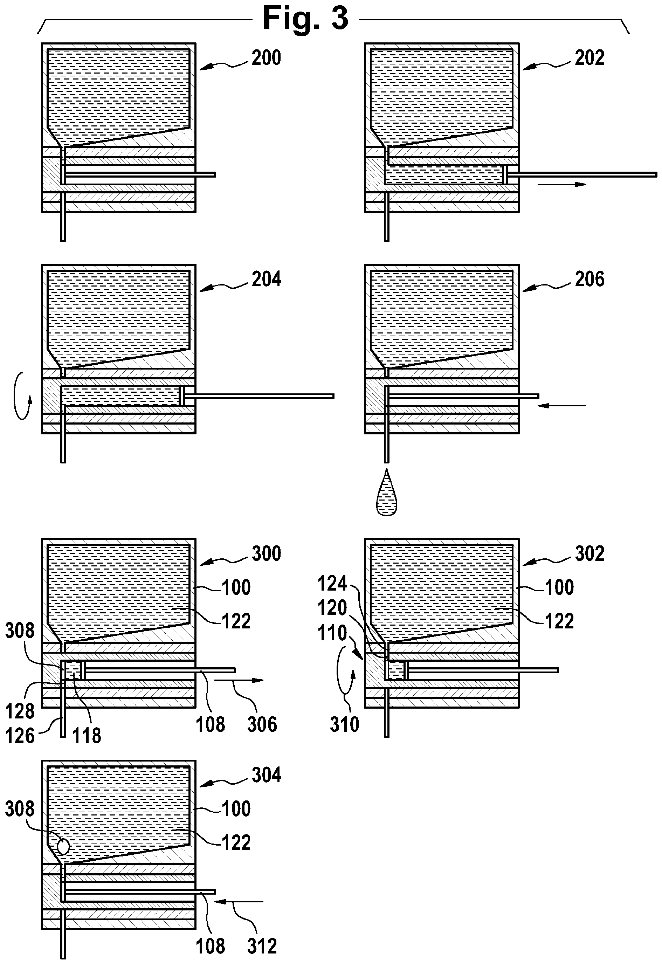

Fig. 3 illustrates a pumping method similar to that shown in Fig. 2 except additional steps are performed to remove fluid from the outlet nozzle 126 and the outlet conduit 128. The same views 202, 204 and 206 are again shown. There are three additional views 300, 302 and 304 of the cartridge 100 are presented. The step according to view 300 is performed after view 206. The plunger 108 is withdrawn in the direction 306 to withdraw fluid from the outlet nozzle 126 in the outlet conduit 128. In this example the plunger 108 is withdrawn enough such that a bubble 208 forms in the pumping chamber 118. Next in view302 the rotary valve 110 is rotated in direction 310 such that the pumping chamber conduit 120 is aligned with the reservoir conduit 124. Finally in view 304 the plunger 108 is depressed in direction 312 thereby forcing fluid out of the pumping chamber 118 into the reservoir 122. In addition the bubble 308 was also forced into the reservoir 122.

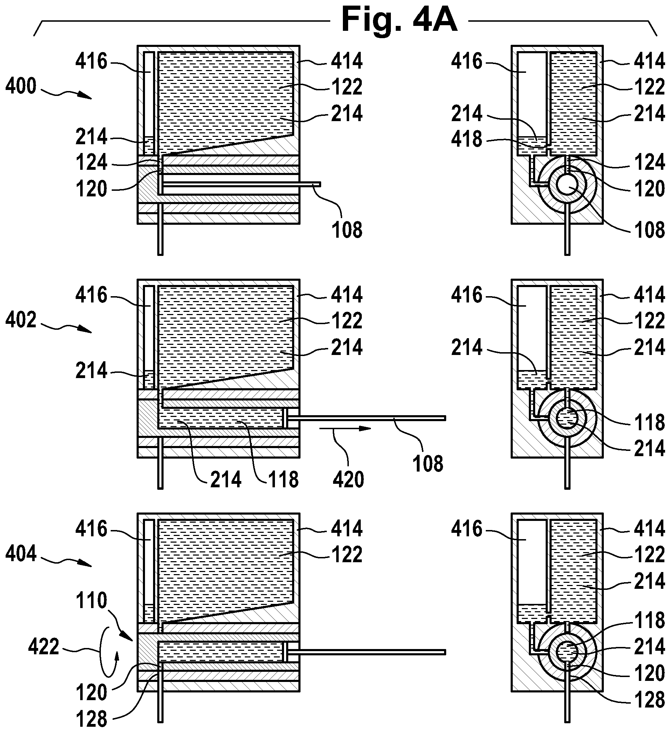

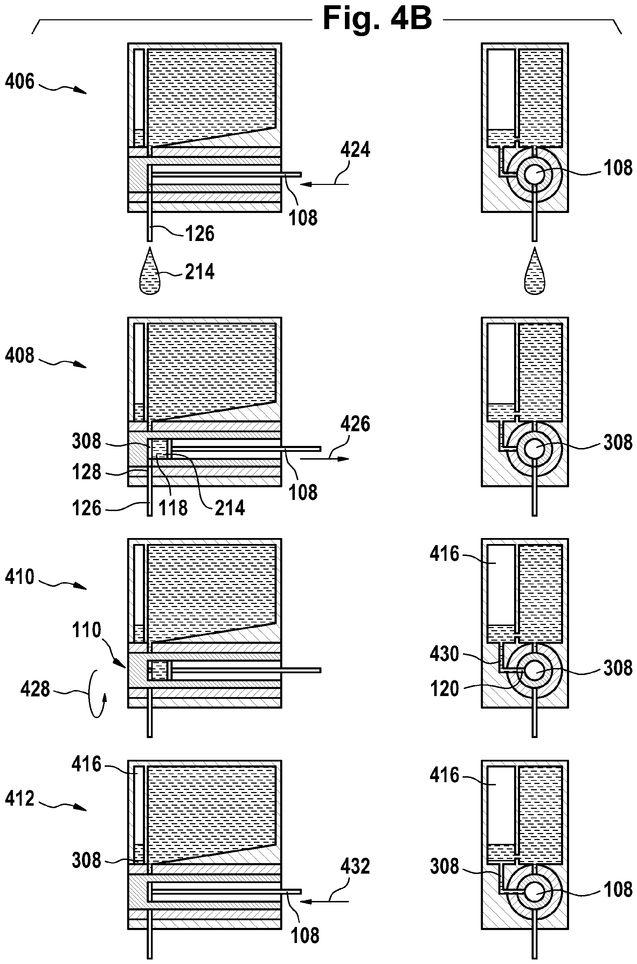

Figs. 4A and 4B shows seven views 400, 402, 404, 406, 408, 410, 412 of a different embodiment of a cartridge 414. In this embodiment the cartridge 414 has a reservoir 122 and a secondary reservoir 416. Fig. 4 illustrates how fluid 214 can be pumped through the outlet conduit 128 where the fluid is taken from the reservoir 122 and then excess fluid from the outlet nozzle 126 and outlet conduit 128 is pumped into the secondary reservoir 416. In this cartridge 414 it can be seen that there is a connecting conduit 418 between the reservoir 122 and the secondary reservoir 416. The connecting conduit 418 is not necessarily present in all embodiments, In some alternative embodiments there is also a membrane which is permeable to the fluid may be placed some place in the connecting conduit 418. View 400 shows the plunger 108 as being fully depressed and the pumping chamber conduit 120 being aligned with the reservoir conduit 124. Next in view 402 the plunger is withdrawn in direction 420 filling the pumping chamber 118 with the fluid 214. Next in view 404 the rotary valve 110 is rotated such that the pumping chamber conduit 120 is aligned with the outlet conduit 128.

The rotary valve is rotated in direction 422. Next inview 406 the plunger 108 is depressed in direction 424 and fluid 214 is forced out of the outlet conduit 128. Next in view 408 the plunger 108 is withdrawn in the direction 426 to withdraw the fluid 214 that was previously in the outlet conduit 128 and the pumping chamber conduit 120 back into the pumping chamber 118. Next in view 410 a rotary valve 110 is rotated in direction 428 to align the pumping chamber conduit 120 with the secondary reservoir conduit 430. Finally in view 412 the plunger 108 is depressed in direction 432 driving the bubble 308 and the fluid 126 into the secondary chamber 416. In some embodiments the secondary chamber 416 may have a vent to atmosphere. In some embodiments the vent may be covered with a filter which allows gas to pass but which keeps the fluid 416 from exiting the cartridge 414. In view 412 the pumping chamber conduit 120 and the secondary reservoir conduit 430 are being shown as being filled with the bubble 308..

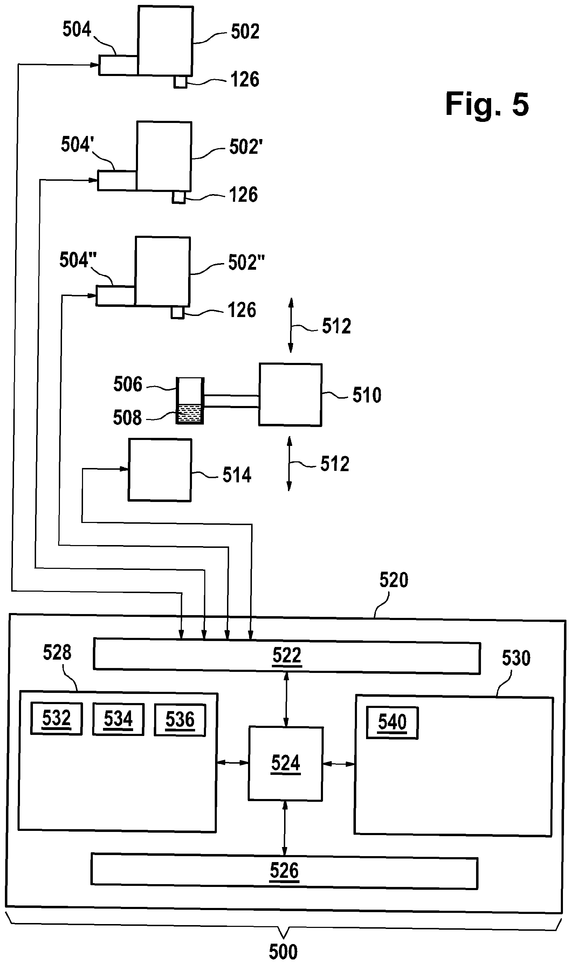

Fig. 5 illustrates an automatic analyzer 500 according to an embodiment of the invention. This automatic analyzer is shown as having three cartridges 502, 502' and 502". There is an actuator assembly 504 connected to cartridge 502. There is an actuator assembly 504' attached to cartridge 502'. There is an actuator assembly 504" attached to cartridge 502". The actuator assemblies 504, 504', 504" are for actuating the rotary valve and plunger of the cartridges 502, 502', 502". The automatic analyzer 500 is shown as having a relative movement means 510 which provides relative movement 512 between a reagent container or cuvette 506 and the cartridges 502, 502' and 502". The reagent container or cuvette 506 is shown as containing a biological sample 508. The cartridges 502, 502', 502" may be used to add one or more fluids to the biological sample 508. The automatic analyzer 500 is shown as further containing a sensor system 514. The sensor system comprises one or more sensors for measuring a quantity or a physical or chemical or biochemical property of the biological sample 508. For example the sensor system 514 may comprise an nuclear magnetic resonance (NMR) system, an optical transmission or reflectance measurement system, a PH meter, a camera system, a polymerase chain reaction (PCR) apparatus, a Electrochemiluminescence (ECL) apparatus, a spectroscopic measurement system, an electrochemical or an optical sensor, and a chromatography system. The relative movement means 510 is also operable for moving the reagent container or cuvette 506 to the sensor system 514.

The arrangement of the cartridges 502, 502', 502" and the sensor system 514 is representative. In some embodiments the reagent container or cuvette 506 may remain in a fixed position and the cartridges 502, 502', 502" may move. The actuation systems 504, 504', 504" and the sensor system 514 are shown as being connected to a hardware interface 522 of a computer system 520. The computer system 520 functions as a controller for the automatic analyzer 500. The computer 520 is further shown as containing a processor 524 which is able to control the operation and function of the automatic analyzer 500 using the hardware interface 522. The processor 524 is shown as further being connected to a user interface 526, computer storage 528 and computer memory 530. The computer storage 528 is shown as containing an analysis request 532. The analysis request 532 contains a request to analyze the biological sample 508.

The computer storage 528 is shown as further containing sensor data 534 received from the sensor system 514. The computer storage 528 is shown as further containing an analysis result 536 which was determined using the sensor data 534. The computer memory 530 contains a control module 540. The control module 540 contains computer executable code which enables the processor 524 to control the operation and function of the automatic analyzer 500. For instance the control module 540 may use the analysis request 532 to generate commands to generate and send to the actuation systems 504, 504', 504", the sensor system 514 and the relative movement system 510. The control module 540 may also generate the analysis result 536 using the sensor data 534.

Various algorithms may be used for controlling the dispensing of the fluid in different embodiments. For instance the actuator assembly may be controlled by the processor to perform a series of predetermined actions to dispense the fluid. In another example a sensor or metering system could be integrated into the automatic analyzer to measure the dispensing of the fluid. In this case a algorithm which uses the actuator assembly and the sensor to form a closed loop feedback to accurately control or meter the dispensing of the fluid.

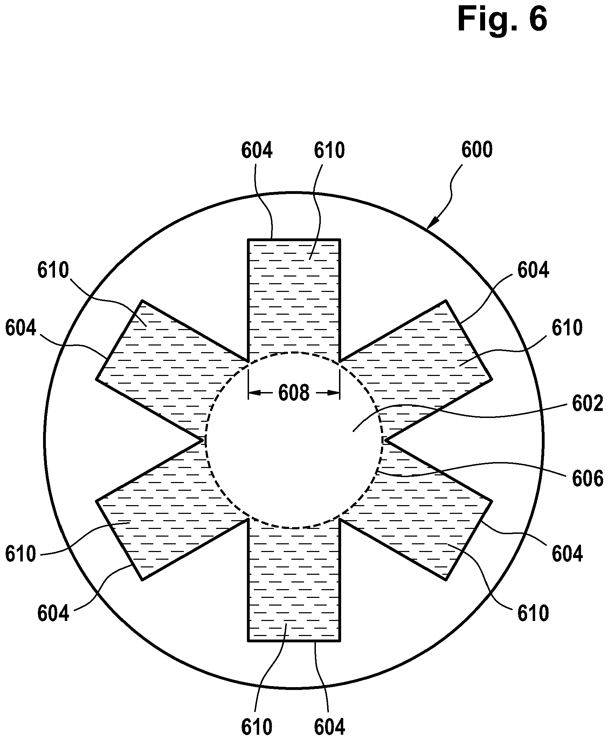

Fig. 6 illustrates a bubble guiding structure 600 according to an embodiment of the invention. The bubble guiding structure 600 may for instance be located within a reservoir or secondary reservoir of a cartridge according to an embodiment of the invention. The bubble guiding structure 600 comprises a bubble channel 602 surrounded by various fluid channels 604. The bubble channel 602 provides a path for a bubble 606. The fluid channels 604 have a space or width 608 which is narrow enough such that the bubble 606 is prevented from entering the fluid channel 604 by the surface tension of the fluid. The bubble channel 602 confines the bubble 606 and allows the bubble to rise while allowing the fluid 610 to go around the bubble.

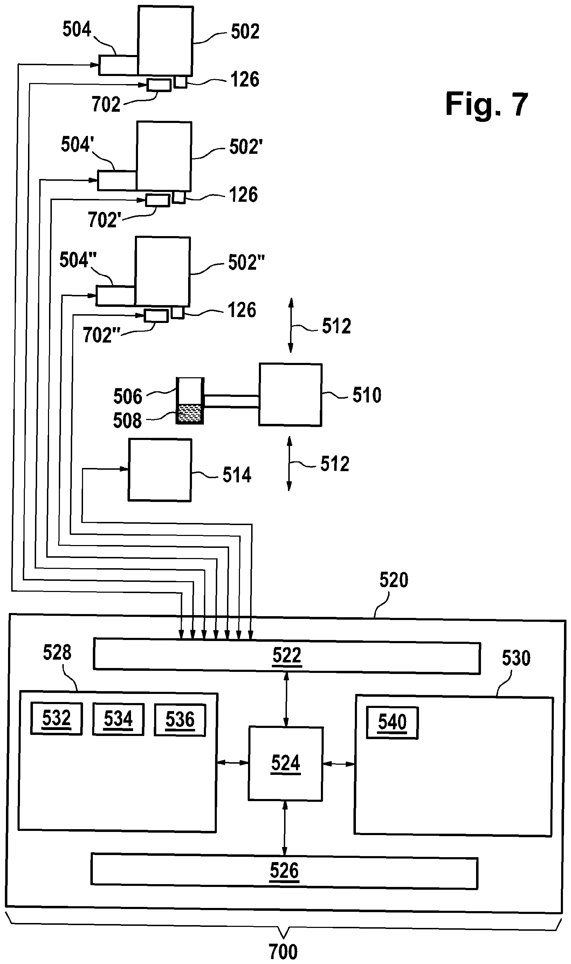

Fig. 7 illustrates a automatic analyzer 700 according to an embodiment of the invention that is similar to the embodiment shown in Fig. 5. The automatic analyzer 700 is similar to the automatic analyzer 500 shown in figure 5. The automatic analyzer 700 of figure 7 additionally has a meniscus detector 702, 702', 702". Each meniscus detector 702, 702', 702" is positioned adjacent to the outlet nozzle 126. The meniscus detector 702, 702', 702" are each connected to the hardware interface 522. This enables the processor 524 to control the actuator assemblies 504, 504', 504" to control the location of the meniscus. This for instance may enable the processor to more accurately and or reproducibly dispense fluid from the cartridges 502, 502', 502".

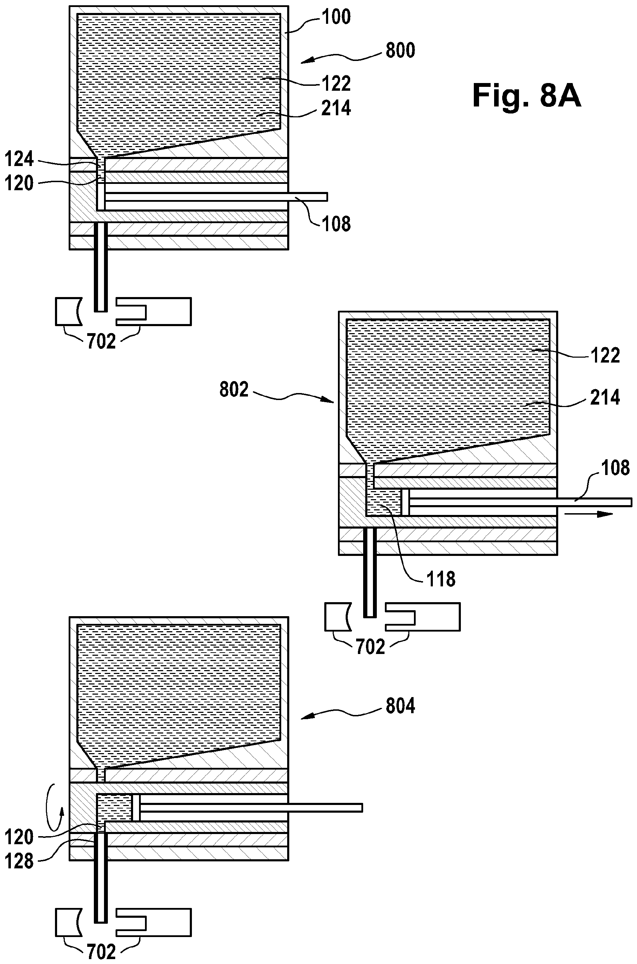

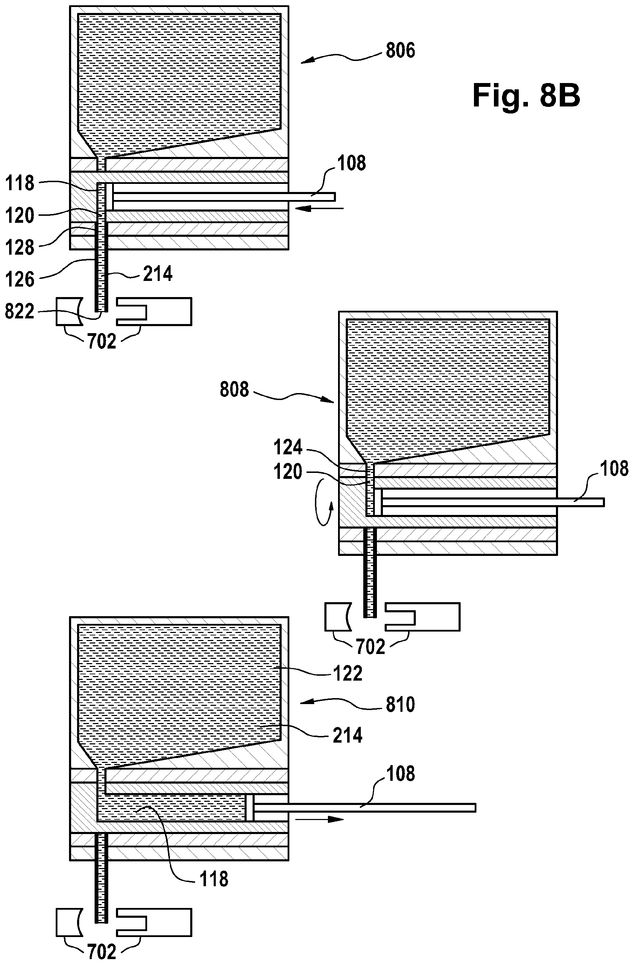

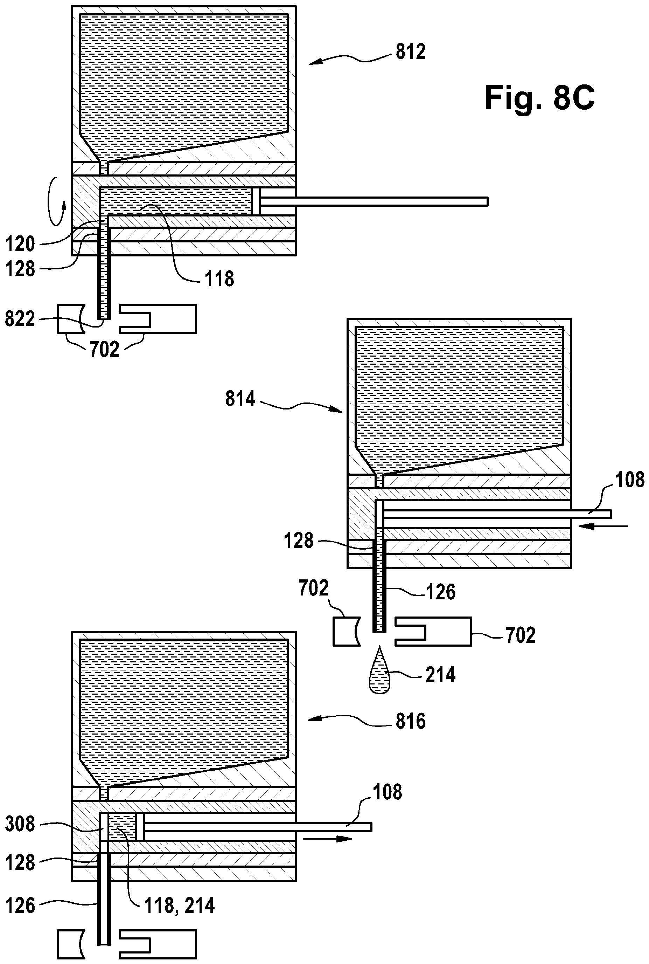

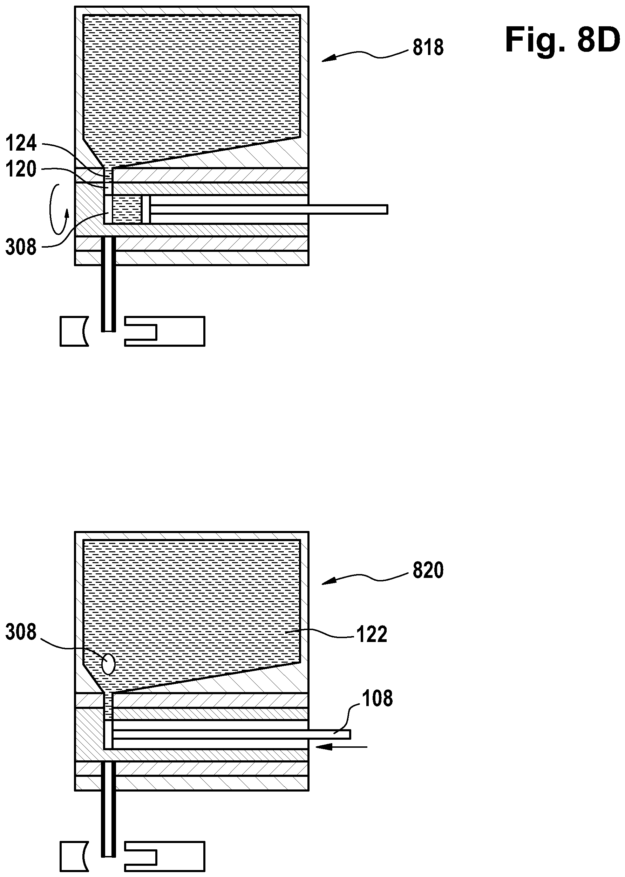

Figure 8 shows 11 views 800, 802, 804, 806, 808, 810, 812, 814, 816, 818, 822 illustrates the functioning of a cartridge 100 in conjunction with a meniscus detector 702. In these examples the meniscus detector 702 is an optical sensor. The use of the optical sensor 702 is exemplary. Other types of sensors may also be used.

In view 800 the pumping chamber conduit 120 has been rotated into position such that it is aligned with the reservoir conduit 124. The plunger 108 is shown in this view 800 as being fully depressed. The pumping chamber 118 is therefore extremely small and is not visible in this view 800. The position of this plunger 108 in this position is not necessarily required as long as the plunger 108 is still able to increase or withdraw a reasonable amount of fluid 214 from the reservoir 122. Next in view 802 the plunger 108 is withdrawn to increase the volume of the pumping chamber 118 and draw fluid 214 from the reservoir 122 into the pumping chamber 118. Next in view 804, the pumping chamber conduit 120 is rotated into position such that is it is aligned with the outlet conduit 128.

In view 806 the plunger 108 is depressed which decreases the volume of the pumping chamber 118. This forces fluid 214 into the outlet conduit 128 and the outlet nozzle 126. The plunger 108 is controlled in accordance with the meniscus detector 702. When the meniscus 822 reached a predetermined position the meniscus detector 702 was used to detect this and the depression of the plunger 108 was halted. Next in view 808 the pumping chamber conduit 120 is again rotated into alignment with the reservoir conduit 124. In view 810 the plunger 108 is withdrawn thereby increasing the volume of the pumping chamber 118 and drying fluid 214 from the reservoir 122. Next in view 812 the pumping chamber conduit 120 is rotated into position so that it is aligned the outlet conduit 128. The pumping chamber 118 is filled with the fluid and the meniscus a 22 is in the predetermined location. Next in view 814 the plunger 108 is depressed forcing fluid out of the outlet nozzle 126. It can be seen in view 814 that there is still fluid within the outlet conduit 128 and the outlet nozzle 126. Next in view 816 the plunger 108 is retracted to withdraw the fluid 214 that was in the outlet conduit 128 and the outlet nozzle 126 back in two the pumping chamber 118 in view 818 the pumping chamber conduit 120 is rotated into position with the reservoir conduit 124. Then finally in view 820 a 20 the plunger 108 is depressed forcing the fluid back into the reservoir 122. A bubble 308 which was inside the pumping chamber is forced out of the pumping chamber and into the reservoir 122.

### List of reference numerals

- 100: cartridge
- 102: actuator assembly
- 104: linear actuator
- 105: direction of linear motion
- 106: rotational actuator
- 107: direction of rotational motion
- 108: plunger
- 110: rotary valve
- 112: cartridge body
- 114: cylindrical portion
- 116: cylindrical space
- 118: pumping chamber
- 120: pumping chamber conduit
- 122: reservoir
- 124: reservoir conduit
- 126: outlet nozzle
- 128: outlet conduit
- 130: coupling assembly
- 200: first view
- 202: second view
- 204: third view
- 206: fourth view
- 208: plunger withdrawn
- 210: rotary valve rotated
- 212: plunger depressed
- 214: fluid
- 300: fifth view
- 302: sixth view
- 304: seventh view
- 306: plunger withdrawn
- 308: bubble
- 310: rotary valve rotated
- 312: plunger depressed
- 400: first view
- 402: second view
- 404: third view
- 406: fourth view
- 408: fifth view
- 410: sixth view
- 412: seventh view
- 414: cartridge
- 416: secondary reservoir
- 418: connecting conduit
- 430: secondary reservoir conduit
- 500: automatic analyzer
- 502: cartridge
- 502': cartridge
- 502": cartridge
- 504: actuator assembly
- 504': actuator assembly
- 504": actuator assembly
- 506: reagent holder or cuvette
- 508: biological sample
- 510: relative movement means
- 512: relative movement
- 514: sensor system
- 520: computer
- 522: hardware interface
- 524: processor
- 526: user interface
- 528: computer storage
- 530: computer memory
- 532: analysis request
- 534: sensor data
- 536: analysis result
- 540: control module
- 600: bubble guiding structure
- 602: bubble channel
- 604: fluid channel
- 606: bubble
- 608: space
- 610: fluid
- 700: automatic analyzer
- 702: meniscus detector
- 800: rotary valve rotated
- 802: plunger withdrawn
- 804: rotary valve rotated
- 806: plunger depressed
- 808: rotary valve rotated
- 810: plunger withdrawn
- 812: rotary valve rotated
- 814: plunger depressed
- 816: plunger withdrawn
- 818: rotary valve rotated
- 820: plunger depressed

## Claims

1. A cartridge (100, 414, 502, 502', 502") for dispensing fluid (214, 610) comprising:
- a cartridge body comprising a cylindrical space (116) and a rotary valve (110), wherein the rotary valve comprises a cylindrical portion (114) adapted to fit into the cylindrical space (116); wherein the cylindrical portion (114) comprises a cavity;
- a reservoir (122) for storing the fluid;
- a reservoir conduit (124) for connecting the reservoir with the rotary valve; and
- an outlet conduit (128) for dispensing the fluid,
**characterized in that**
the cartridge body comprises a plunger (108), wherein the rotary valve comprises a pumping chamber (118) for pumping the fluid, wherein the pumping chamber comprises a pumping chamber conduit (120) which is formed in a wall of the cylindrical portion (114), wherein the rotary valve is operable for rotating said pumping chamber conduit (120), wherein the pumping chamber conduit is connected with the pumping chamber, wherein the pumping chamber is formed by the cavity and the plunger; wherein the plunger (108) is operable for changing the volume of the pumping chamber, wherein the rotary valve and the plunger are operable for being actuated independently,- wherein the rotary valve is operable for rotating the pumping chamber conduit to connect with the reservoir conduit, wherein the rotary valve is further operable for rotating the pumping chamber conduit to connect with the outlet conduit.

2. The cartridge of claim 1, wherein the cartridge further comprises a return conduit connected to the reservoir, wherein the pumping chamber conduit is operable for receiving fluid from a first portion of the reservoir, wherein the return conduit is operable for returning fluid to a second portion of the reservoir, wherein the rotary valve is further operable for rotating the pumping chamber conduit to connect to the return conduit.

3. The cartridge of claim 1 or 2, wherein the cartridge further comprises:
- a secondary reservoir (416); and
- a secondary reservoir conduit (430), wherein the rotary valve is further operable for rotating the pumping chamber conduit to connect to the secondary reservoir conduit.

4. The cartridge of claim 3, wherein the cartridge further comprises a connecting conduit (418), wherein the connecting conduit is operable for transporting fluid between the secondary reservoir and the reservoir.

5. The cartridge of claim 4, wherein cartridge comprises a membrane blocking the connecting conduit, and wherein the membrane is permeable to the fluid.

6. The cartridge of claim 4 or 5, wherein the secondary reservoir comprises a bubble guiding structure (600).

7. The cartridge of claim 4, 5, or 6, wherein the secondary reservoir comprises a vent, wherein the vent is sealed with a filter, wherein the filter is permeable to air, and wherein the filter is operable for sealing the fluid in the cartridge.

8. The cartridge of any one of the preceding claims, wherein the fluid comprises any one of the following: magnetic beads, latex beads, a dispersion, nanoparticles, a blood grouping reagent, an immune reagent, an antibody, an enzyme, a recombinant protein, a virus isolate, a virus, a biological reagent, solvent, diluent, a protein, a salt, a detergent, a nucleic acid, an acid, a base, and combinations thereof.

9. The cartridge of any one of the preceding claims, wherein the cartridge further comprises a sensor operable for metering fluid dispensed by the outlet nozzle.

10. The cartridge of any one of the preceding claims, wherein the cartridge further comprises a coupling assembly (130) for attaching the rotary valve and the plunger to an actuator assembly.

11. An automatic analyzer (500) for analyzing a biological sample, wherein the automatic analyzer is operable for holding a cartridge (100, 414, 502, 502', 502") according to any one of claims 1 through 10, wherein the automatic analyzer comprises:
- an actuator assembly (504, 504', 504") operable for separate linear actuation (105, 208, 212, 306, 312, 420, 424, 426, 432) of the plunger and for rotational actuation (107, 210, 310, 422, 428) of the rotary valve;
- a controller (520) for controlling the operation of the actuator assembly.

12. The automatic analyzer of claim 11, wherein the controller is operable for:
- controlling (200) the actuator assembly to rotate the pumping chamber conduit to connect with the reservoir conduit by rotating the rotary valve;
- controlling (202) the actuator assembly to fill the pumping chamber by increasing (208) the volume of the pumping chamber with the plunger;
- controlling (204) the actuator assembly to rotate (210) the pumping chamber conduit to connect with the outlet conduit by rotating the rotary valve; and
- controlling (206) the actuator assembly to pump (212) the fluid through the outlet conduit by decreasing the volume of the pumping chamber with the plunger.

13. The automatic analyzer of claim 12 or 11, wherein the controller is operable for controlling (300) the actuator assembly to retrieve the fluid from the outlet conduit by increasing (306) the volume of the pumping chamber with the plunger.

14. The automatic analyzer of any one of claims 11 through 13, wherein the controller is operable for:
- controlling (302) the actuator assembly to rotate (310) the pumping chamber conduit to connect with the reservoir conduit by rotating the rotary valve; and
- controlling (304) the actuator assembly to return the fluid to the reservoir by decreasing (312) the volume of the pumping chamber with the plunger.

15. The automatic analyzer of any one of claims 11 through 14, wherein the controller is operable for:
- controlling the actuator assembly to rotate the pumping chamber conduit to connect with the reservoir conduit by rotating the rotary valve; and
- controlling the actuator assembly to mix the fluid in the reservoir by repeatedly increasing and decreasing the volume of the pumping chamber with the plunger.

16. The automatic analyzer of any one of claims 11 through 15, wherein the cartridge comprises an outlet nozzle, wherein the automatic analyzer further comprises a meniscus detector (702, 702', 702") for detecting a meniscus of the fluid, wherein the controller is operable for:
- controlling the actuator to force fluid through the outlet nozzle;
- detecting the meniscus using the meniscus detector; and
- controlling the actuator to halt the forcing of fluid through the outlet when the meniscus is in a predetermined location.

17. The automatic analyzer of any one of claims 11 through 16, wherein the automatic analyzer is operable to hold multiple cartridges (504, 504', 504"), wherein each of the multiple cartridges is according to any one of claims 1 through 11.

18. A method of operating a cartridge according to any one of claims 1 through 10, wherein the cylindrical portion (114) is configured to be rotated within the cylindrical space (116) such that:
- the pumping chamber conduit (120) can be connected with the reservoir conduit (124) thereby closing the outlet conduit (128) by the cylindrical portion and can be connected with the outlet conduit (128) thereby closing the reservoir conduit (124) by the cylindrical portion (124); and
- the plunger (128) being configured to be actuated in a linear direction such that the volume of the pumping chamber can be increased to fill the pumping chamber and decreased to pump the fluid through the outlet conduit;
wherein the method comprises the steps of:
- rotating the cylindrical portion of the rotary valve to rotate the pumping chamber conduit to connect with the reservoir conduit;
- increasing the volume of the pumping chamber with the plunger to fill the pumping chamber;
- rotating the cylindrical portion of the rotary valve to rotate the pumping chamber conduit to connect with the outlet conduit; and
- decreasing the volume of the pumping chamber with the plunger to pump the fluid through the outlet conduit.

## Patentansprüche

1. Kartusche (100, 414, 502, 502', 502") zur Ausgabe einer Flüssigkeit (214, 610), umfassend:
- einen Kartuschenkörper, umfassend einen zylindrischen Raum (116) und ein Drehventil (110), wobei das Drehventil einen zylindrischen Abschnitt (114) umfasst, der dazu ausgelegt ist, in den zylindrischen Raum (116) zu passen; wobei der zylindrische Abschnitt (114) einen Hohlraum umfasst;
- ein Reservoir (122) zum Speichern der Flüssigkeit;
- eine Reservoirleitung (124) zum Verbinden des Reservoirs mit dem Drehventil; und
- eine Auslassleitung (128) zur Ausgabe der Flüssigkeit,
**dadurch gekennzeichnet, dass**
der Kartuschenkörper einen Kolben (108) umfasst, wobei das Drehventil eine Pumpkammer (118) zum Pumpen der Flüssigkeit umfasst, wobei die Pumpkammer eine Pumpkammerleitung (120) umfasst, die in einer Wand des zylindrischen Abschnitts (114) gebildet ist, wobei das Drehventil einsetzbar ist, die Pumpkammerleitung (120) zu drehen, wobei die Pumpkammerleitung mit der Pumpkammer verbunden ist; wobei die Pumpkammer durch den Hohlraum und den Kolben gebildet ist; wobei der Kolben (108) einsetzbar ist, das Volumen der Pumpkammer zu ändern, wobei das Drehventil und der Kolben einsetzbar sind, unabhängig voneinander betätigt zu werden, - wobei das Drehventil einsetzbar ist, die Pumpkammerleitung zu drehen, um mit der Pumpkammer verbunden zu werden, wobei das Drehventil ferner einsetzbar ist, die Pumpkammerleitung zu drehen, um mit der Auslassleitung verbunden zu werden.

2. Kartusche nach Anspruch 1, wobei die Kartusche ferner eine mit dem Reservoir verbundene Rückführleitung umfasst, wobei die Pumpkammerleitung einsetzbar ist, Flüssigkeit von einem ersten Abschnitt des Reservoirs aufzunehmen, wobei die Rückführleitung einsetzbar ist, Flüssigkeit zu einem zweiten Abschnitt des Reservoirs zurückzuführen, wobei das Drehventil ferner einsetzbar ist, die Pumpkammerleitung zu drehen, um mit der Rückführleitung verbunden zu werden.

3. Kartusche nach Anspruch 1 oder 2, wobei die Kartusche ferner umfasst:
- ein Sekundärreservoir (416); und
- eine Sekundärreservoirleitung (430), wobei das Drehventil ferner einsetzbar ist, die Pumpkammerleitung zu drehen, um mit der Sekundärreservoirleitung verbunden zu werden.

4. Kartusche nach Anspruch 3, wobei die Kartusche ferner eine Verbindungsleitung (418) umfasst, wobei die Verbindungsleitung einsetzbar ist, Flüssigkeit zwischen dem Sekundärreservoir und dem Reservoir zu transportieren.

5. Kartusche nach Anspruch 4, wobei die Kartusche eine Membran umfasst, die die Verbindungsleitung blockiert, und wobei die Membran für die Flüssigkeit durchlässig ist.

6. Kartusche nach Anspruch 4 oder 5, wobei das Sekundärreservoir eine Blasenführungsstruktur (600) umfasst.

7. Kartusche nach Anspruch 4, 5 oder 6, wobei das Sekundärreservoir eine Entlüftung umfasst, wobei die Entlüftung durch einen Filter abgedichtet ist, wobei der Filter für Luft durchlässig ist, und wobei der Filter einsetzbar ist, die Flüssigkeit in der Kartusche abzudichten.

8. Kartusche nach einem der vorhergehenden Ansprüche, wobei die Flüssigkeit eines der Folgenden umfasst:
Magnetkügelchen, Latexkügelchen, eine Dispersion, Nanopartikel, ein Reagens zu Blutgruppenbestimmung, ein Immunreagens, einen Antikörper, ein Enzym, ein rekombinantes Protein, ein Virusisolat, einen Virus, ein biologisches Reagens, Lösungsmittel, Verdünnungsmittel, ein Protein, ein Salz, ein Detergent, eine Nukleinsäure, eine Säure, eine Base und Kombinationen davon.

9. Kartusche nach einem der vorhergehenden Ansprüche, wobei die Kartusche ferner einen Sensor umfasst, der einsetzbar ist, durch die Auslassdüse ausgegebene Flüssigkeit zu dosieren.

10. Kartusche nach einem der vorhergehenden Ansprüche, wobei die Kartusche ferner eine Kupplungsanordnung (130) zum Befestigen des Drehventils und des Kolbens an einer Aktuatoranordnung umfasst.

11. Automatischer Analysator (500) zum Analysieren einer biologischen Probe, wobei der automatische Analysator einsetzbar ist, eine Kartusche (100, 414, 502, 502', 502") nach einem der Ansprüche 1 bis 10 zu halten, wobei der automatische Analysator Folgendes umfasst:
- eine Aktuatoranordnung (504, 504', 504"), die zur separaten linearen Betätigung (105, 208, 212, 306, 312, 420, 424, 426, 432) des Kolbens und zur Drehbetätigung (107, 210, 310, 422, 428) des Drehventils einsetzbar ist;
- eine Steuerung (520) zum Steuern des Betriebs der Aktuatoranordnung.

12. Automatischer Analysator nach Anspruch 11, wobei die Steuerung einsetzbar ist:
- die Aktuatoranordnung zu steuern (200), um die Pumpkammerleitung zu drehen, um durch Drehen des Drehventils mit der Reservoirleitung verbunden zu werden;
- die Aktuatoranordnung zu steuern (202), um die Pumpkammer durch Erhöhen (208) des Volumens der Pumpkammer mit dem Kolben zu füllen;
- die Aktuatoranordnung zu steuern (204), um die Pumpkammerleitung zu drehen (210), um durch Drehen des Drehventils mit der Auslassleitung verbunden zu werden; und
- die Aktuatoranordnung zu steuern (206), um die Flüssigkeit durch Verringern des Volumens der Pumpkammer mit dem Kolben durch die Auslassleitung zu pumpen (212).

13. Automatischer Analysator nach Anspruch 12 oder 11, wobei die Steuerung einsetzbar ist, die Aktuatoranordnung zu steuern (300), um die Flüssigkeit aus der Auslassleitung durch Erhöhen (306) des Volumens der Pumpkammer mit dem Kolben zurückzugewinnen.

14. Automatischer Analysator nach einem der Ansprüche 11 bis 13, wobei die Steuerung einsetzbar ist:
- die Aktuatoranordnung zu steuern (302), um die Pumpkammerleitung zu drehen (310), um durch Drehen des Drehventils mit der Reservoirleitung verbunden zu werden; und
- die Aktuatoranordnung zu steuern (304), um die Flüssigkeit durch Verringern (312) des Volumens der Pumpkammer mit dem Kolben zu dem Reservoir zurückzuführen.

15. Automatischer Analysator nach einem der Ansprüche 11 bis 14, wobei die Steuerung einsetzbar ist:
- die Aktuatoranordnung zu steuern, um die Pumpkammerleitung zu drehen, um durch Drehen des Drehventils mit der Reservoirleitung verbunden zu werden; und
- die Aktuatoranordnung zu steuern, um die Flüssigkeit in dem Reservoir durch wiederholtes Erhöhen und Verringern des Volumens der Pumpkammer mit dem Kolben zu mischen.

16. Automatischer Analysator nach einem der Ansprüche 11 bis 15, wobei die Kartusche eine Auslassdüse umfasst, wobei der automatische Analysator ferner einen Meniskusdetektor (702, 702', 702") zum Erfassen eines Meniskus der Flüssigkeit umfasst, wobei die Steuerung einsetzbar ist:
- den Aktuator zu steuern, um Flüssigkeit durch die Auslassdüse zu pressen;
- den Meniskus unter Verwendung des Meniskusdetektors zu erfassen; und
- den Aktuator zu steuern, um das Pressen von Flüssigkeit durch den Auslass zu stoppen, wenn sich der Meniskus an einer vorbestimmten Stelle befindet.

17. Automatischer Analysator nach einem der Ansprüche 11 bis 16, wobei der automatische Analysator einsetzbar ist, mehrere Kartuschen (504, 504', 504") zu halten, wobei jede der mehreren Kartuschen nach einem der Ansprüche 1 bis 11 vorliegt.

18. Verfahren zum Einsetzen einer Kartusche nach einem der Ansprüche 1 bis 10, wobei der zylindrische Abschnitt (114) konfiguriert ist, in dem zylindrischen Raum (116) gedreht zu werden, sodass:
- die Pumpkammerleitung (120) mit der Reservoirleitung (124) verbunden werden kann, wodurch die Auslassleitung (128) durch den zylindrischen Abschnitt geschlossen wird und mit der Auslassleitung (128) verbunden werden kann, wodurch die Reservoirleitung (124) durch den zylindrischen Abschnitt (124) geschlossen wird; und
- der Kolben (128) konfiguriert ist, in einer linearen Richtung betätigt zu werden, sodass das Volumen der Pumpkammer erhöht werden kann, um die Pumpkammer zu füllen und verringert werden kann, um die Flüssigkeit durch die Auslassleitung zu pumpen;
wobei das Verfahren folgende Schritte umfasst:
- Drehen des zylindrischen Abschnitts des Drehventils, um die Pumpkammerleitung zu drehen, um mit der Reservoirleitung verbunden zu werden;
- Erhöhen des Volumens der Pumpkammer mit dem Kolben, um die Pumpkammer zu füllen;
- Drehen des zylindrischen Abschnitts des Drehventils, um die Pumpkammerleitung zu drehen, um mit der Auslassleitung verbunden zu werden; und
- Verringern des Volumens der Pumpkammer mit dem Kolben, um die Flüssigkeit durch die Auslassleitung zu pumpen.

## Revendications

1. Cartouche (100, 414, 502, 502', 502") pour distribuer un fluide (214, 610) comprenant :
- un corps de cartouche comprenant un espace cylindrique (116) et une vanne rotative (110), dans laquelle la vanne rotative comprend une partie cylindrique (114) conçue pour entrer dans l'espace cylindrique (116) ; dans laquelle la partie cylindrique (114) comprend une cavité ;
- un réservoir (122) pour stocker le fluide ;
- un conduit de réservoir (124) pour relier le réservoir à la vanne rotative ; et
- un conduit de sortie (128) pour distribuer le fluide ;
**caractérisé en ce que**
le corps de cartouche comprend un plongeur (108), dans laquelle la vanne rotative comprend une chambre de pompage (118) pour pomper le fluide, dans laquelle la chambre de pompage comprend un conduit de chambre de pompage (120) qui est formé dans une paroi de la partie cylindrique (114), dans laquelle la vanne rotative peut être utilisée pour faire pivoter ledit conduit de chambre de pompage (120), dans laquelle le conduit de chambre de pompage est relié à la chambre de pompage, dans laquelle la chambre de pompage est formée par la cavité et le plongeur ; dans laquelle le plongeur (108) peut être utilisé pour modifier le volume de la chambre de pompage, dans laquelle la vanne rotative et le plongeur peuvent être activés de manière indépendante,- dans laquelle la vanne rotative peut être utilisée pour faire pivoter le conduit de chambre de pompage pour le relier au conduit de réservoir, dans laquelle la vanne rotative peut en outre être utilisée pour faire pivoter le conduit de chambre de pompage pour le relier au conduit de sortie.

2. Cartouche selon la revendication 1, dans laquelle la cartouche comprend en outre un conduit de retour relié au réservoir, dans laquelle le conduit de chambre de pompage peut être utilisé pour recevoir le fluide provenant d'une première partie du réservoir, dans laquelle le conduit de retour peut être utilisé pour renvoyer le fluide vers une seconde partie du réservoir, dans laquelle la vanne rotative peut en outre être utilisée pour faire pivoter le conduit de chambre de pompage pour le relier au conduit de retour.

3. Cartouche selon la revendication 1 ou 2, dans laquelle la cartouche comprend en outre :
- un réservoir secondaire (416) ; et
- un conduit de réservoir secondaire (430), dans laquelle la vanne rotative peut en outre être utilisée pour faire pivoter le conduit de chambre de pompage pour le relier au conduit de réservoir secondaire.

4. Cartouche selon la revendication 3, dans laquelle la cartouche comprend en outre un conduit de raccordement (418), dans laquelle le conduit de raccordement peut être utilisé pour transporter le fluide entre le réservoir secondaire et le réservoir.

5. Cartouche selon la revendication 4, dans laquelle la cartouche comprend une membrane bloquant le conduit de raccordement et dans laquelle la membrane est perméable au fluide.

6. Cartouche selon la revendication 4 ou 5, dans laquelle le réservoir secondaire comprend une structure de guidage de bulle (600).

7. Cartouche selon la revendication 4, 5 ou 6, dans laquelle le réservoir secondaire comprend un évent, dans laquelle l'évent est scellé à l'aide d'un filtre, dans laquelle le filtre est perméable à l'air et dans laquelle le filtre peut être utilisé pour sceller le fluide dans la cartouche.

8. Cartouche selon l'une quelconque des revendications précédentes, dans laquelle le fluide comprend l'un quelconque des éléments suivants : des perles magnétiques, des perles de latex, une dispersion, des nanoparticules, un réactif de groupage sanguin, un réactif immun, un anticorps, une enzyme, une protéine recombinante, un isolat de virus, un virus, un réactif biologique, un solvant, un diluant, une protéine, un sel, un détergent, un acide nucléique, un acide, une base et des combinaisons de ceux-ci.

9. Cartouche selon l'une quelconque des revendications précédentes, dans laquelle la cartouche comprend en outre un détecteur pouvant être utilisé pour mesurer le fluide distribué par la buse de sortie.

10. Cartouche selon l'une quelconque des revendications précédentes, dans laquelle la cartouche comprend en outre un ensemble couplage (130) pour fixer la vanne rotative et le plongeur à un ensemble actionneur.

11. Analyseur automatique (500) pour analyser un échantillon biologique, dans lequel l'analyseur biologique peut être utilisé pour contenir une cartouche (100, 414, 502, 502', 502") selon l'une quelconque des revendications 1 à 10, dans lequel l'analyseur automatique comprend :
- un ensemble actionneur (504, 504', 504") pouvant être utilisé pour un actionnement linéaire séparé (105, 208, 212, 306, 312, 420, 424, 426, 432) du plongeur et pour un actionnement rotatif (107, 210, 310, 422, 428) de la vanne rotative ;
- un dispositif de commande (520) pour commander le fonctionnement de l'ensemble actionneur.

12. Analyseur automatique selon la revendication 11, dans lequel le dispositif de commande peut être utilisé pour :
- commander (200) l'ensemble actionneur pour faire pivoter le conduit de chambre de pompage pour le relier au conduit de réservoir en faisant pivoter la vanne rotative ;
- commander (202) l'ensemble actionneur pour remplir la chambre de pompage en augmentant (208) le volume de la chambre de pompage avec le plongeur;
- commander (204) l'ensemble actionneur pour faire pivoter (210) le conduit de chambre de pompage pour le relier au conduit de sortie en faisant pivoter la vanne rotative ; et
- commander (206) l'ensemble actionneur pour pomper (212) le fluide à travers le conduit de sortie en diminuant le volume de la chambre de pompage avec le plongeur.

13. Analyseur automatique selon la revendication 12 ou 11, dans lequel le dispositif de commande peut être utilisé pour commander (300) l'ensemble actionneur pour récupérer le fluide du conduit de sortie en augmentant (306) le volume de la chambre de pompage avec le plongeur.

14. Analyseur automatique selon l'une quelconque des revendications 11 à 13, dans lequel le dispositif de commande peut être utilisé pour :
- commander (302) l'ensemble actionneur pour faire pivoter (310) le conduit de chambre de pompage pour le relier au conduit de réservoir en faisant pivoter la vanne rotative ; et
- commander (304) l'ensemble actionneur pour renvoyer le fluide vers le réservoir en diminuant (312) le volume de la chambre de pompage avec le plongeur.

15. Analyseur automatique selon l'une quelconque des revendications 11 à 14, dans lequel le dispositif de commande peut être utilisé pour :
- commander l'ensemble actionneur pour faire pivoter le conduit de chambre de pompage pour le relier au conduit de réservoir en faisant pivoter la vanne rotative ; et
- commander l'ensemble actionneur pour mélanger le fluide dans le réservoir en augmentant et en diminuant de manière répétée le volume de la chambre de pompage avec le plongeur.

16. Analyseur automatique selon l'une quelconque des revendications 11 à 15, dans lequel la cartouche comprend une buse de sortie, dans lequel l'analyseur automatique comprend en outre un détecteur de ménisque (702, 702', 702") pour détecter un ménisque du fluide, dans lequel le dispositif de commande peut être utilisé pour :
- commander l'actionneur pour forcer le fluide à travers la buse de sortie ;
- détecter le ménisque à l'aide du détecteur de ménisque ; et
- commander l'actionneur pour arrêter le forçage du fluide à travers la sortie lorsque le ménisque est dans un emplacement prédéterminé.

17. Analyseur automatique selon l'une quelconque des revendications 11 à 16, dans lequel l'analyseur automatique peut être utilisé pour contenir de multiples cartouches (504, 504', 504"), dans lequel chacune des multiples cartouches est selon l'une quelconque des revendications 1 à 11.

18. Procédé d'utilisation d'une cartouche selon l'une quelconque des revendications 1 à 10, dans lequel la partie cylindrique (114) est configurée pour pivoter à l'intérieur de l'espace cylindrique (116) de sorte que :
- le conduit de chambre de pompage (120) peut être relié au conduit de réservoir (124) fermant ainsi le conduit de sortie (128) par la partie cylindrique et peut être relié au conduit de sortie (128) fermant ainsi le conduit de réservoir (124) par la partie cylindrique (124) ; et
- le plongeur (128) étant configuré pour être actionné dans une direction linéaire de sorte que le volume de la chambre de pompage peut être augmenté pour remplir la chambre de pompage et diminué pour pomper le fluide à travers le conduit de sortie ;
dans lequel le procédé comprend les étapes :
- de rotation de la partie cylindrique de la vanne rotative pour faire pivoter le conduit de chambre de pompage pour le relier au conduit de réservoir ;
- d'augmentation du volume de la chambre de pompage avec le plongeur pour remplir la chambre de pompage ;
- de rotation de la partie cylindrique de la vanne rotative pour faire pivoter le conduit de chambre de pompage pour le relier au conduit de sortie ; et
- de diminution du volume de la chambre de pompage avec le plongeur pour pomper le fluide à travers le conduit de sortie.
